# EUROPEAN PATENT APPLICATION

(11) **EP 4 548 963 A1**
(43) Date of publication of application: **07.05.2025**
(21) Application number: 23849971.9
(22) Date of filing: 26.07.2023
(51) Int. Cl.: A61N 1/36, A61B 18/12

(54) **HIGH-FREQUENCY TREATMENT DEVICE**

(30) Priority: 02.08.2022 JP 2022123372
(71) Applicant: Kabushiki Kaisha Top, Adachi-ku, Tokyo 120-0035 (JP)
(72) Inventor: IKEUCHI Atsushi, Tokyo 120-0035 (JP)
(74) Representative: Schön, Christoph
(86) International application number: PCT/JP2023/027322
(87) International publication number: WO 2024/029416

(57) **Abstract**

There is provided a high-frequency treatment device capable of stimulating a nerve of a patient or the like while avoiding an unexpected influence on the patient or the like. In a case where a setting operation of a target current value and an output start operation are performed through an operation unit 16 (input interface) in a stimulation mode, a main control unit 141 controls an output unit 110 to start output of a nerve stimulation pulse signal and to match a current value of the nerve stimulation pulse signal with the target current value. On the other hand, in a case where an output stop operation is performed through the operation unit 16, the main control unit 141 causes the output unit 110 to stop the output of the nerve stimulation pulse signal. During a period in which the nerve stimulation pulse signal is output by the output unit 110, a change in the target current value through the operation unit 16 is prohibited.

## Description

### Technical Field

The present invention relates to a high-frequency treatment device having a function of applying a nerve stimulation pulse signal to a treatment target such as a human or an animal.

### Background Art

In the related art, a nerve stimulation pulse signal is output from an electrode disposed inside a body of a patient or the like (a human and other animals), and a treatment of stimulating a nerve of the patient or the like is performed in the medical field (for example, see Patent Literature 1).

### Citation List

### Patent Literature

Patent Literature 1: PCT Patent Publication No. 2017-093865

### Summary of Invention

### Technical Problem

However, if a current value of the nerve stimulation pulse signal is arbitrarily changed while the signal is being output from the electrode disposed inside the body of the patient or the like, there is a possibility that an unexpected influence will be exerted on the patient or the like.

Therefore, an object of the present invention is to provide a high-frequency treatment device capable of stimulating a nerve of a patient or the like while avoiding an unexpected influence on the patient or the like.

### Solution to Problem

A high-frequency treatment device according to the present invention includes: a plurality of electrode connection terminals; an output unit configured to output a nerve stimulation pulse signal in a stimulation mode and output a high-frequency signal in a coagulation mode between a plurality of electrodes connected to the plurality of electrode connection terminals, respectively; an input interface through which a switching operation between the stimulation mode and the coagulation mode, a setting operation of a target current value, an output start operation, and an output stop operation are able to be performed; and a control unit configured to cause the output unit to start outputting the nerve stimulation pulse signal and control a current value of the nerve stimulation pulse signal to match the target current value in a case where the setting operation of the target current value and the output start operation are performed through the input interface in the stimulation mode, and cause the output unit to stop outputting the nerve stimulation pulse signal in a case where the output stop operation is performed through the input interface, and a change in the target current value through the input interface is prohibited during a period in which the nerve stimulation pulse signal is output by the output unit.

### Brief Description of Drawings

FIG. 1 is a schematic explanatory diagram related to a configuration of a high-frequency treatment device as an embodiment of the present invention.
FIG. 2 is a block diagram representing functional elements of the high-frequency treatment device in FIG. 1.
FIG. 3 is an explanatory view related to a nerve stimulation function of the high-frequency treatment device.
FIG. 4 is an explanatory diagram related to a nerve blocking function of the high-frequency treatment device.
FIG. 5 is an explanatory diagram related to an impedance measurement function of the high-frequency treatment device.

### Description of Embodiments

### (Configuration)

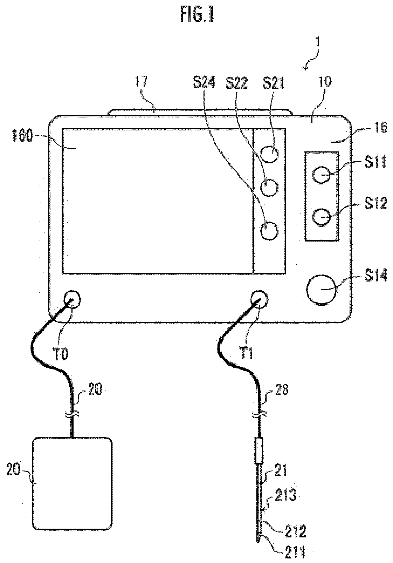

A high-frequency treatment device 1 according to an embodiment of the present invention illustrated in FIG. 1 is a device for performing nerve blocking by stimulating a nerve with a nerve stimulation pulse signal and partially heating a peripheral nerve with a high-frequency signal. A scheme of the nerve blocking may be any of a high-frequency thermal coagulation method or a pulse high-frequency method. The "high-frequency thermal coagulation method" is a method of blocking pain signals by heating a part of a nerve tissue at a temperature of about 80°C for several minutes by a high frequency current flowing from electrodes to thermally coagulate the nerve tissue. The "pulse high-frequency method" is a method of blocking pain signals without damaging a nerve by causing a high-frequency current to intermittently flow through a part of the nerve tissue to heat the nerve tissue at a temperature of 42°C or lower for ten and several minutes.

As illustrated in FIG. 1, the high-frequency treatment device 1 includes a main body 10 (a housing or a casing), a main electrode 21 connected to the main body 10, and a counter electrode plate 22 (electrode) connected to the main body 10. The main electrode 21 and/or the counter electrode plate 22 may not be a component of the high-frequency treatment device 1 (or may be an additional component).

The main body 10 accommodates or supports components, which will be described below. A main electrode connector T1 to which the main electrode 21 is electrically connected and a counter electrode plate connector T0 to which the counter electrode plate 22 is electrically connected are provided on a lower portion of the front surface of the main body 10.

A touch panel type and/or pressing type operation unit 16 (constituting an "input interface" of the present invention) is provided on an upper portion of the front surface of the main body 10. The operation unit 16 is configured of a touch panel display 160 that receives input operations such as various settings and displays various types of information, an ON button S11 that receives an output start operation (treatment start operation), an OFF button S12 that receives an output stop operation (treatment end operation), a control knob S14 for adjusting and setting output power (for example, a current), a stimulation mode key S21, a coagulation mode key S22, and an impedance measurement key S24. For example, a target current value of the nerve stimulation pulse signal can be adjusted or set in increments of 0.5 mA through the control knob S14. A target voltage of the high-frequency voltage to be applied between the main electrode 21 and the counter electrode plate 22 can be set through the operation unit 16.

After the target output (the target current value and/or the target voltage value) is set, a rotation operation of the control knob S14 may be received as an output start operation (treatment start operation) in addition to the operation of the ON button S11. If the ON button S11 (auto start key) is broken, the treatment can be continued by the output start operation function of the control knob S14 even in a situation where the patient has already undergone craniotomy. In addition, a protection function using software is added to a main control unit 141 and/or a sub-control unit 142 such that the set current value is not exceeded regardless of how many times the control knob S14 is rotated.

When the stimulation mode key S21 is pressed, the high-frequency treatment device 1 is switched to a "stimulation mode", and a state where the nerve stimulation pulse signal can be output from the main electrode 21 is achieved. If the coagulation mode key S22 is pressed, the high-frequency treatment device 1 is switched to a "coagulation mode", and a state where a high-frequency signal can be output from the main electrode 21 is achieved. When the impedance measurement key S24 is pressed, the high-frequency treatment device 1 is switched to an "impedance measurement mode", and a state where the impedance between the main electrode 21 and the counter electrode plate 22 is measured is achieved.

A "menu key" may be provided in addition to the stimulation mode key S21, the coagulation mode key S22, and the impedance measurement key S24. When the menu key is pressed, a menu screen is displayed on the touch panel display 160, and it is possible to check an energized state of the main electrode 21 and/or the counter electrode plate 22 (disconnection check) and to perform setting and the like of the type and volume of a buzzer sound output from an acoustic output device (not illustrated) such as a speaker and screen brightness. When the menu key is pressed after the transition to the stimulation mode or the coagulation mode, the display screen on the touch panel display 160 can be transitioned to the menu screen.

A top portion of the main body 10 is provided with a handle 17 for carrying the main body 10. A rear surface of the main body 10 is provided with a power connector and a power switch that are connected to a commercial AC power supply to receive power supply (not illustrated).

The main electrode 21 is configured to be inserted into the inside of the human body or the like and to apply each of a nerve stimulation pulse signal and a high-frequency signal to the human body. In the present embodiment, the main electrode 21 is configured to have a substantially cylindrical needle shape that can puncture a human body or the like. In another embodiment, the main electrode 21 is configured to be a tubular needle capable of puncturing a human body or the like and capable of putting a drug or the like into the human body or the like via the lumen of the main electrode 21. The main electrode 21 is an insulating portion 212 in which most of a distal end portion excluding the non-insulating portion 211 is insulated with an insulating coating, and each of the nerve stimulation pulse signal and the high-frequency signal is output from the non-insulating portion 211.

The main electrode 21 includes, for example, a thermocouple 213 for measuring a treatment temperature provided at a distal end portion of the lumen or the inner space. The main electrode 21 may have a shape other than the above-described shape, and for example, may have a rod shape that is inserted into a needle tube or a catheter. In addition, the thermocouple 213 may be provided separately from the main electrode 21.

The counter electrode plate 22 is a flat plate-shaped electrode that is disposed by being attached to a skin surface of a human body or the like, which is a treatment target, and is used for allowing a nerve stimulation pulse signal and a high-frequency signal to flow between itself and the main electrode 21 inserted into the human body or the like. In other words, the counter electrode plate 22 is used in a case where a high-frequency current is caused to flow in a so-called monopolar manner. The counter electrode plate 22 is electrically connected to the main body 10 via a counter electrode plate cable 20 and the counter electrode plate connector T0. Note that although the counter electrode plate 22 is configured in a rectangular flat plate shape in the present embodiment, the counter electrode plate 22 may have another shape.

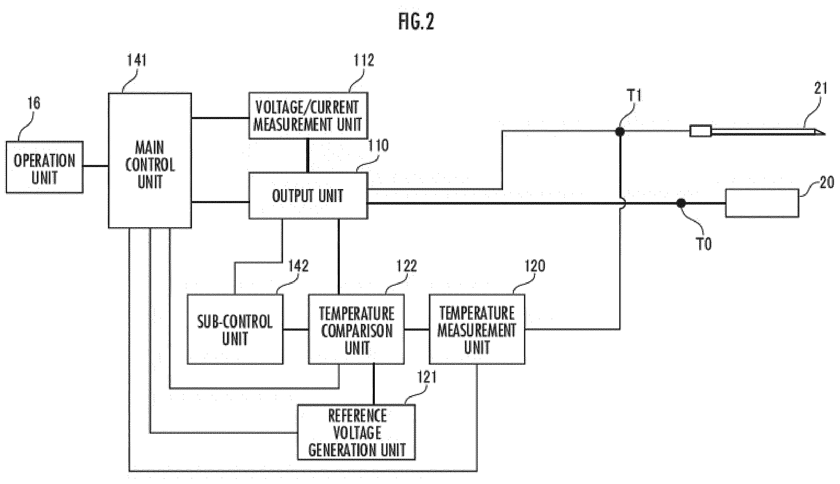

As illustrated in FIG. 2, the high-frequency treatment device 1 includes, as functional elements, an output unit 110, a voltage/current measurement unit 112, a temperature measurement unit 120, a reference voltage generation unit 121, a temperature comparison unit 122, a main control unit 141, and a sub-control unit 142. The main control unit 141 and/or the sub-control unit 142 constitute a "control unit" in the present invention.

The output unit 110 is configured to generate high-frequency power (primary high-frequency power signal) of a voltage based on a preset frequency (470 to 490 kHz, for example) and an output control signal transmitted from the main control unit 141 based on power supplied from a commercial AC power supply and output pulse high-frequency power generated by turning ON/OFF the primary high-frequency power based on a pulse signal (a pulse width wp and a pulse period Tp) from the main control unit 141. In addition, the output unit 110 is configured to generate continuous high-frequency power in a case where the pulse signal from the main control unit 141 is always ON.

The output unit 110 is connected to the main electrode connector T1 and the counter electrode plate connector T0, and is thus connected to the main electrode 21 and the counter electrode plate 22 so as to be able to output each of the nerve stimulation signal and the high-frequency signal thereto. The output unit 110 is configured of a known circuit including a transformer, whereby a human body or the like, which is a treatment target, is insulated from the commercial AC power supply.

The voltage/current measurement unit 112 is configured of a known circuit or the like and is configured to measure the high-frequency current and the voltage output from the output unit 110. The voltage/current measurement unit 112 is configured to generate a voltage measurement signal corresponding to the high-frequency voltage generated by the output unit 110 and a current measurement signal corresponding to the high-frequency current and transmit the signals to the main control unit 141.

The temperature measurement unit 120 is configured of a known circuit or the like and is configured to generate a temperature measurement signal (for example, 25 mV/°C) based on a signal received from the thermocouple 213 and transmit the temperature measurement signal to each of the main control unit 141 and the temperature comparison unit 122.

The reference voltage generation unit 121 is configured of a known circuit or the like and is configured to receive a target temperature transmitted from the main control unit 141, generate a reference voltage corresponding to the target temperature, and then transmit the reference voltage to the temperature comparison unit 122.

The temperature comparison unit 122 is configured of a known circuit or the like and is configured to receive the reference voltage transmitted from the reference voltage generation unit 121 and the measured temperature signal transmitted from the temperature measurement unit 120, compare the target temperature corresponding to the reference voltage with the measured temperature corresponding to the measured temperature signal, and transmit the temperature comparison result (differential voltage signal) to the main control unit 141 and/or the sub-control unit 142.

The main control unit 141 is configured of a computing processing element, such as a CPU, a storage element, such as a ROM and/or a RAM, an I/O circuit, and the like. The main control unit 141 is configured to control operations of the components of the high-frequency treatment device 1, such as the operation unit 16 and the output unit 110, to execute the high-frequency treatment. The main control unit 141 measures the voltage, the current, the power, and the impedance based on the voltage measurement signal and the current measurement signal received from the voltage/current measurement unit 112 and causes the touch panel display 160 to display the measured values along with the measured temperature. The main control unit 141 is configured to control the high-frequency power output from the output unit 110 such that the measured voltage corresponding to the voltage measurement signal received from the voltage/current measurement unit 112 matches the target voltage in a case where the target voltage has been set through the operation unit 16.

The sub-control unit 142 is configured of a computing processing element such as a CPU, a storage element such as a ROM and/or a RAM, and an I/O circuit, similarly to the main control unit 141. The sub-control unit 142 may be configured of a known pulse width modulation (PWM) circuit or the like instead of a microcomputer. The sub-control unit 142 compares the temperature comparison result (differential voltage signal) transmitted from the temperature comparison unit 122 with a sawtooth wave signal to generate a pulse signal and transmits the pulse signal to the output unit 110.

The voltage/current measurement unit 112, the temperature measurement unit 120, the reference voltage generation unit 121, the temperature comparison unit 122, and the sub-control unit 142 are insulated from a commercial AC power supply in order to protect a human body or the like as a treatment target.

### (Functions)

According to the high-frequency treatment device 1 with the above-described configuration, the main control unit 141 (or the main control unit 141 and the sub-control unit 142) executes processing as described below.

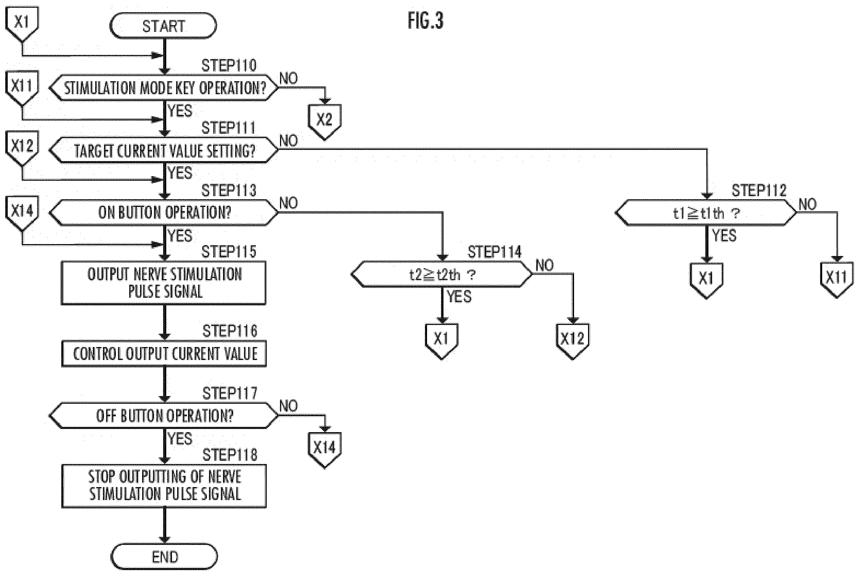

First, whether or not the stimulation mode key S21 has been operated is determined (FIG. 3/STEP 110). In a case where it is determined that the stimulation mode key S21 has been operated (FIG. 3/YES in STEP 110), whether or not the target current value has been set through the touch panel display 160 and/or the control knob S14 is determined (FIG. 3/STEP 111). In a case where it is determined that the target current value has not been set (FIG. 3/NO in STEP 111), whether or not an elapsed time t₁ after the operation of the stimulation mode key S21 is equal to or greater than a first designated time t₁ₜₕ is determined (FIG. 3/STEP 112). In a case where it is determined that the elapsed time t₁ is less than the first designated time t₁ₜₕ (FIG. 3/NO in STEP 112), the determination processing as to whether or not the target current value has been set is repeated (FIG. 3/STEP 111). In a case where it is determined that the elapsed time t₁ is equal to or greater than the first designated time t₁ₜₕ (FIG. 3/YES in STEP 112), whether or not the stimulation mode key S21 has been operated is determined again (FIG. 3/STEP 110). The determination processing as to whether or not the elapsed time t₁ after the operation of the stimulation mode key S21 is equal to or greater than the first designated time t₁ₜₕ (FIG. 3/STEP 112) may be omitted, and the determination processing as to whether or not the target current value has been set may be repeated (FIG. 3/STEP 111) until it is determined that the target current value has been set (FIG. 3/YES in STEP 111).

In a case where it is determined that the target current value has been set (FIG. 3/YES in STEP 111), whether or not the ON button S11 has been operated (whether or not the output start operation has been performed) is determined (FIG. 3/STEP 113). The target current value is output to the touch panel display 160. In a case where it is determined that the ON button S11 has not been operated (FIG. 3/NO in STEP 113), whether or not an elapsed time t₂ after the setting of the target current value is equal to or greater than a second designated time t₂ₜₕ is determined (FIG. 3/STEP 114). The first designated time t₁ₜₕ and the second designated time t₂ₜₕ may be the same, and one of the first designated time t₁ₜₕ or the second designated time t₂ₜₕ may be set to be longer than the other.

In a case where it is determined that the elapsed time t₂ is less than the second designated time t₂ₜₕ (FIG. 3/NO in STEP 114), the determination processing as to whether or not the ON button S11 has been operated is repeated (FIG. 3/STEP 113). In a case where it is determined that the elapsed time t₂ is equal to or greater than the second designated time t₂ₜₕ (FIG. 3/YES in STEP 114), whether or not the stimulation mode key S21 has been operated is determined again (FIG. 3/STEP 110). The determination processing as to whether or not the elapsed time t₂ after the setting of the target current value is equal to or greater than the second designated time t₂ₜₕ (FIG. 3/STEP 114) may be omitted, and the determination processing as to whether or not the ON button S11 has been operated may be repeated (FIG. 3/STEP 113) until it is determined that the ON button S11 has been operated (FIG. 3/YES in STEP 113).

In a case where it is determined that the ON button S11 has been operated (FIG. 3/YES in STEP 113), the nerve stimulation pulse signal is output from the main electrode 21 via the output unit 110 (FIG. 3/STEP 115). In addition to the case where it is determined that the ON button S11 has been operated, the nerve stimulation pulse signal may be output from the main electrode 21 via the output unit 110 in a case where it is determined that the control knob S14 has been rotated. The output current value is controlled such that the output current value of the current flowing between the main electrode 21 and the counter electrode plate 20 measured through the voltage/current measurement unit 112 matches the target current value (or such that an error from the target current value is equal to or less than a threshold value) (FIG. 3/STEP 116). In this case, a change in the target current value is prohibited even if the touch panel display 160 and/or the control knob S14 is operated.

Although the target current value is confirmed in response to the operation of the ON button S11 in the present embodiment, the target current value may be confirmed in response to another operation such as a touch operation of a button or a key displayed on the touch panel display 160, or the target current value may be confirmed in response to the fact that a certain time has elapsed since the target current value was last set, in other embodiments.

Subsequently, whether or not the OFF button S12 has been operated (whether or not the output stop operation has been performed) is determined (FIG. 3/STEP 117). In a case where it is determined that the OFF button S12 has not been operated (FIG. 3/NO in STEP 117), the nerve stimulation pulse signal is continuously output from the main electrode 21 via the output unit 110 (FIG. 3/STEP 115). On the other hand, in a case where it is determined that the OFF button S12 has been operated (FIG. 3/YES in STEP 117), the output of the nerve stimulation pulse signal from the main electrode 21 via the output unit 110 is stopped (FIG. 3/STEP 118).

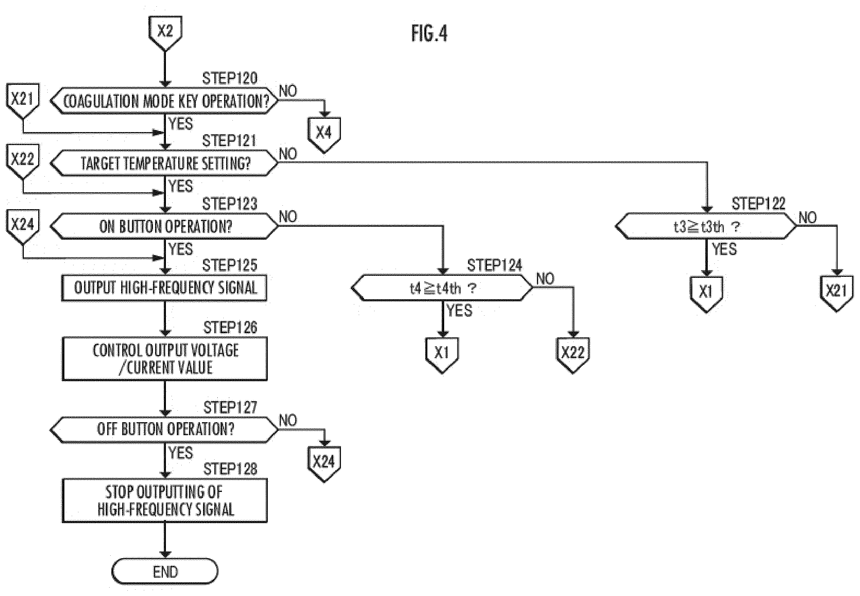

In a case where it is determined that the stimulation mode key S21 has not been operated (FIG. 3/NO in STEP 110), whether or not the coagulation mode key S22 has been operated is determined (FIG. 4/STEP 120). In a case where it is determined that the coagulation mode key S22 has been operated (FIG. 4/YES in STEP 120), whether or not the target temperature has been set through the touch panel display 160 and/or the control knob S14 is determined (FIG. 4/STEP 121). In a case where it is determined that the target temperature has not been set (FIG. 4/NO in STEP 121), whether or not an elapsed time t₃ from the operation of the coagulation mode key S22 is equal to or greater than a third designated time t₃ₜₕ is determined (FIG. 4/STEP 122). In a case where it is determined that the elapsed time t₃ is less than the third designated time t₃ₜₕ (FIG. 4/NO in STEP 122), the determination processing as to whether or not the target voltage has been set is repeated (FIG. 4/STEP 121). In a case where it is determined that the elapsed time t₃ is equal to or greater than the third designated time t₃ₜₕ (FIG. 4/YES in STEP 122), whether or not the stimulation mode key S21 has been operated is determined again (FIG. 3/STEP 110). The determination processing as to whether or not the elapsed time t₃ after the operation of the coagulation mode key S22 is equal to or greater than the third designated time t₃ₜₕ (FIG. 4/STEP 122) may be omitted, and the determination processing as to whether or not the target temperature has been set (FIG. 4/YES in STEP 121) may be repeated until it is determined that the target temperature has been set (FIG. 4/YES in STEP 121).

In a case where it is determined that the target temperature has been set (FIG. 4/YES in STEP 121), whether or not the ON button S11 has been operated is determined (FIG. 4/STEP 123). In a case where it is determined that the ON button S11 has not been operated (FIG. 4/NO in STEP 123), whether or not an elapsed time t₄ after the setting of the target temperature is equal to or greater than a fourth designated time t₄ₜₕ is determined (FIG. 4/STEP 124). The third designated time t₃ₜₕ and the fourth designated time t₄ₜₕ may be the same, and one of the third designated time t₃ₜₕ and the fourth designated time t₄ₜₕ may be set to be longer than the other. The first designated time t₁ₜₕ, the second designated time t₂ₜₕ, the third designated time t₃ₜₕ, and the fourth designated time t₄ₜₕ may be the same as each other or may be set to be different from each other.

In a case where it is determined that the elapsed time t₄ is less than the fourth designated time t₄ₜₕ (FIG. 4/NO in STEP 124), the determination processing as to whether or not the ON button S11 has been operated is repeated (FIG. 4/STEP 123). In a case where it is determined that the elapsed time t₄ is equal to or greater than the fourth designated time t₄ₜₕ (FIG. 4/YES in STEP 124), whether or not the stimulation mode key S21 has been operated is determined again (FIG. 4/STEP 120). The determination processing (FIG. 4/STEP 124) as to whether or not the elapsed time t₄ after the target temperature is set is equal to or greater than the fourth designated time t₄ₜₕ may be omitted, and the determination processing as to whether or not the ON button S11 has been operated (FIG. 4/YES in STEP 123) may be repeated until it is determined that the ON button S11 has been operated (FIG. 4/YES in STEP 123).

In a case where it is determined that the ON button S11 has been operated (FIG. 4/YES in STEP 123), a high-frequency signal (or a high-frequency pulse signal) is output from the main electrode 21 via the output unit 110 (FIG. 4/STEP 125). In addition to the case where it is determined that the ON button S11 has been operated, in a case where it is determined that the control knob S14 has been rotated, the high-frequency signal (or the high-frequency pulse signal) may be output from the main electrode 21 via the output unit 110. The output voltage and/or the output current value is controlled such that the temperature of the main electrode 21 measured through the temperature measurement unit 120 matches the target temperature (or an error from the target temperature is equal to or less than a threshold value) (FIG. 4/STEP 126).

In the coagulation mode, the output voltage is controlled based on the measured temperature, such that the temperature of the main electrode 21 or the periphery thereof is controlled to be stabilized at the set temperature or in the vicinity of the set temperature. In addition to the control of the output voltage using the temperature measured once, a temperature obtained by performing moving average (sixteen times) is used for a temperature graph representing the numerical value of the measured temperature and the temporal change thereof, which are to be displayed on the touch panel display 160. When the temperature is measured, a slight variation occurs in the measurement result for each measurement due to influences of noise and the like even if the output voltage is constant. Therefore, when the measurement result for each time is displayed on the touch panel display 160, the numerical value of the temperature may frequently change, which may cause a user to feel uneasy. Therefore, arrangement is made to further stabilize the numerical value by adopting the averaged measured temperature and to make the user feel safe. According to the specification, the influence of noise is compressed to about 1/4 by adopting the sixteen-time moving average.

Although the target temperature is confirmed in response to the operation of the ON button S11 in the present embodiment, the target temperature may be confirmed in response to other operations such as a touch operation of a button or a key displayed on the touch panel display 160, and the target temperature may be confirmed in response to elapse of a specific time after the target temperature is finally set, in other embodiments.

Subsequently, whether or not the OFF button S12 has been operated is determined (FIG. 4/STEP 127). In a case where it is determined that the OFF button S12 has not been operated (FIG. 4/NO in STEP 127), the high-frequency signal is continuously output from the main electrode 21 via the output unit 110 (FIG. 4/STEP 125). On the other hand, in a case where it is determined that the OFF button S12 has been operated (FIG. 4/YES in STEP 127), the output of the high-frequency signal from the main electrode 21 is stopped via the output unit 110 (FIG. 4/STEP 128). The output of the high-frequency signal may be stopped in response to the fact that the cumulative output time (for example, a point in time at which the measured temperature reaches the target temperature may be used as a starting point) of the high-frequency signal has reached a predetermined time or the cumulative number of times of output of the high-frequency pulse signal (for example, this may be a cumulative number after the measured temperature reaches the target temperature) has reached a predetermined number of times, instead of the fact that the OFF button S12 has been operated.

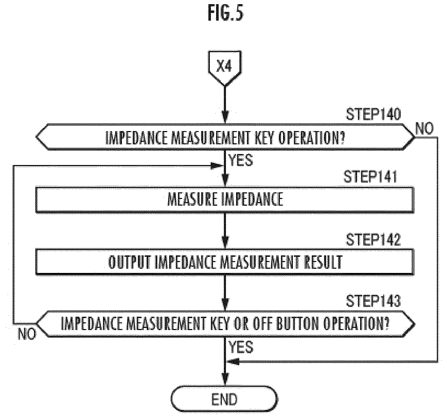

In a case where it is determined that the coagulation mode key S22 has not been operated (FIG. 4/NO in STEP 120), whether or not the impedance measurement key S24 has been operated is determined (FIG. 5/STEP 140). In a case where it is determined that the impedance measurement key S24 has been operated (FIG. 5/YES in STEP 140), the voltage/current measurement unit 112 measures the impedance between the main electrode 21 and the counter electrode plate 20 (FIG. 5/STEP 141). Then, the measurement result of the impedance is output to the touch panel display 160 (FIG. 5/STEP 142). Furthermore, whether or not the impedance measurement key S24 or the OFF button S12 has been operated is determined (FIG. 5/STEP 143). In a case where it is determined that the impedance measurement key S24 or the OFF button S12 has not been operated (FIG. 5/NO in STEP 143), the measurement processing of the impedance between the main electrode 21 and the counter electrode plate 20 is repeated (FIG. 5/STEP 141).

In a case where it is determined that the impedance measurement key S24 or the OFF button S12 has been operated (FIG. 5/YES in STEP 143), the series of processing ends. In a case where it is determined that the impedance measurement key S24 has not been operated (FIG. 5/NO in STEP 140), the series of processing ends. Then, the processing of the determination processing as to whether or not the stimulation mode key S21 has been operated (FIG. 3/STEP 110) and the subsequent processing are repeated.

### (Other embodiments of present invention)

Although the high-frequency treatment device 1 includes a single main electrode connector T1 corresponding to the main electrode which is a single electrode in the aforementioned embodiment, the high-frequency treatment device 1 may include two or more electrode connectors corresponding to two or more electrodes (excluding the counter electrode plate 20).

After the output of the high-frequency signal from the main electrode 21 is stopped via the output unit 110 (see FIG. 4/STEP 128), the main control unit 141 may determine whether or not the temperature of the main electrode 21 or the periphery thereof is equal to or less than a predetermined temperature (for example, 38°C) based on the temperature measurement signal received from the temperature measurement unit 120, and in a case where the determination result is positive, a beep sound may be output from the acoustic output device. Instead of or in addition to the beep sound, a designated image (a message or the like) may be displayed on the touch panel display 160.

The beep sound and/or the message can notify an operator of the timing of pulling out the main electrode 21 from the inside of the body of the patient in view of the temperature of the main electrode 21 or the periphery thereof.

While the designated output time of the high-frequency signal in the coagulation mode is set through the operation unit 16, and the high-frequency signal is output from the main electrode 21 by the main control unit 141, countdown from the designated output time may be performed through a timer, and the output of the high-frequency signal may be automatically stopped in a case where a remaining cauterization time as a result of the countdown becomes zero (in a case where the cumulative output time of the high-frequency signal has reached the designated output time). A temperature graph representing a temporal change in the temperature of the main electrode 21 or the periphery thereof may be displayed on the touch panel display 160 while being sequentially updated by the main control unit 141.

When the high-frequency signal is output from the main electrode 21 and the OFF button S12 (or the stop key) is operated once in the coagulation mode of the high-frequency treatment device 1, the output of the high-frequency signal from the main electrode 21 may be temporarily stopped without resetting the remaining cauterization time during which the high-frequency signal is output by the main control unit 141 to 0. In this case, the temperature graph displayed on the touch panel display 160 may be continuously updated. Thereafter, in a case where the ON button S11 (or an auto start key) is operated, the output of the high-frequency signal from the main electrode 21 is restarted by the main control unit 141, and the output of the high-frequency signal is continued until the remaining cauterization time becomes zero. In a case where the OFF button S12 (or the stop key) is operated again in the temporarily stopped state of the output of the high-frequency signal, the main control unit 141 resets the remaining time, and a completely stopped state where the updating of the temperature graph displayed on the touch panel display 160 is also stopped is achieved.

If the output of the high-frequency signal is completely stopped as it is when a need to stop the output of the high-frequency signal arises for some reason, the timer is reset, and the operator needs to reset the timer to the initial remaining cauterization time and to restart the output of the high-frequency signal. However, the temporary stop function enables the operator to selectively execute the stop and the restart of the output of the high-frequency signal without resetting the timer.

### Description of Reference Numerals

1: high-frequency treatment device
16: operation unit (input interface)
20: counter electrode plate (electrode)
21: main electrode
110: output unit
112: voltage/current measurement unit
120: temperature measurement unit
121: reference voltage generation unit
122: temperature comparison unit
141: main control unit
142: sub-control unit

## Claims

1. A high-frequency treatment device comprising:
a plurality of electrode connection terminals;
an output unit configured to output a nerve stimulation pulse signal in a stimulation mode and output a high-frequency signal in a coagulation mode between a plurality of electrodes connected to the plurality of electrode connection terminals, respectively;
an input interface through which a switching operation between the stimulation mode and the coagulation mode, a setting operation of a target current value, an output start operation, and an output stop operation are able to be performed; and
a control unit configured to cause the output unit to start outputting the nerve stimulation pulse signal and control a current value of the nerve stimulation pulse signal to match the target current value in a case where the setting operation of the target current value and the output start operation are performed through the input interface in the stimulation mode, and cause the output unit to stop outputting the nerve stimulation pulse signal in a case where the output stop operation is performed through the input interface,
wherein a change in the target current value through the input interface is prohibited during a period in which the nerve stimulation pulse signal is output by the output unit.

2. The high-frequency treatment device according to Claim 1,
wherein an impedance measurement start operation and an impedance measurement stop operation are able to be performed through the input interface, and
the control unit starts impedance measurement between the electrodes by causing a current to flow between the plurality of electrodes in a case where the impedance measurement start operation is performed through the input interface, and stops impedance measurement between the electrodes by stopping the current flowing between the plurality of electrodes in a case where the impedance measurement stop operation is performed through the input interface.

3. The high-frequency treatment device according to Claim 1, wherein, in at least one of cases where the output of the high-frequency signal in the coagulation mode is stopped, and in a case where a temperature of at least one electrode among the plurality of electrodes or a periphery of the at least one electrode is equal to or less than a predetermined temperature, an auditory or visual notification indicating the temperature being equal to or less than the predetermined temperature is provided.

4. The high-frequency treatment device according to Claim 1,
wherein a designated output time of the high-frequency signal in the coagulation mode is set through the input interface,
the output of the high-frequency signal is automatically stopped in a case where a cumulative output time of the high-frequency signal reaches the designated output time, and
the cumulative output time of the high-frequency signal until the output of the high-frequency signal is stopped is saved in a case where the output of the high-frequency signal is stopped before the cumulative output time of the high-frequency signal reaches the designated output time, and in a case where the output of the high-frequency signal is restarted thereafter, the cumulative output time of the high-frequency signal is added to the stored cumulative output time.

5. The high-frequency treatment device according to Claim 4, wherein in a case where the output of the high-frequency signal is stopped before the cumulative output time of the high-frequency signal reaches the designated output time, and further, a stop instruction is issued, the cumulative output time of the high-frequency signal until the stop instruction is issued is reset to 0.

6. The high-frequency treatment device according to Claim 1, further comprising:
the plurality of electrodes.

## Amended claims

### Amended claims under Art. 19.1 PCT

1. [Amended] A high-frequency treatment device comprising:
a plurality of electrode connection terminals;
an output unit configured to output a nerve stimulation pulse signal in a stimulation mode and output a high-frequency signal in a coagulation mode between a plurality of electrodes connected to the plurality of electrode connection terminals, respectively;
an input interface that includes, as separate operators, a switching operator for switching between the stimulation mode and the coagulation mode, a setting operator for setting a target current value, an ON operator for starting output, and an OFF operator for stopping the output; and
a control unit configured to cause the output unit to start outputting the nerve stimulation pulse signal and control a current value of the nerve stimulation pulse signal to match the target current value in a case where a setting operation of the target current value is performed through the setting operator and an output start operation is performed through the ON operator in the stimulation mode, and cause the output unit to stop outputting the nerve stimulation pulse signal in a case where the output stop operation is performed through the OFF operator,
wherein a change in the target current value through the input interface is prohibited during a period from the output start operation through the ON operator to the output stop operation through the OFF operator in the stimulation mode.

2. The high-frequency treatment device according to Claim 1,
wherein an impedance measurement start operation and an impedance measurement stop operation are able to be performed through the input interface, and
the control unit starts impedance measurement between the electrodes by causing a current to flow between the plurality of electrodes in a case where the impedance measurement start operation is performed through the input interface, and stops impedance measurement between the electrodes by stopping the current flowing between the plurality of electrodes in a case where the impedance measurement stop operation is performed through the input interface.

3. The high-frequency treatment device according to Claim 1, wherein, in at least one of cases where the output of the high-frequency signal in the coagulation mode is stopped, and in a case where a temperature of at least one electrode among the plurality of electrodes or a periphery of the at least one electrode is equal to or less than a predetermined temperature, an auditory or visual notification indicating the temperature being equal to or less than the predetermined temperature is provided.

4. The high-frequency treatment device according to Claim 1,
wherein a designated output time of the high-frequency signal in the coagulation mode is set through the input interface,
the output of the high-frequency signal is automatically stopped in a case where a cumulative output time of the high-frequency signal reaches the designated output time, and
the cumulative output time of the high-frequency signal until the output of the high-frequency signal is stopped is saved in a case where the output of the high-frequency signal is stopped before the cumulative output time of the high-frequency signal reaches the designated output time, and in a case where the output of the high-frequency signal is restarted thereafter, the cumulative output time of the high-frequency signal is added to the stored cumulative output time.

5. The high-frequency treatment device according to Claim 4, wherein in a case where the output of the high-frequency signal is stopped before the cumulative output time of the high-frequency signal reaches the designated output time, and further, a stop instruction is issued, the cumulative output time of the high-frequency signal until the stop instruction is issued is reset to 0.

6. The high-frequency treatment device according to Claim 1, further comprising:
the plurality of electrodes.

Statement under Art. 19.1 PCT

Basis of amendment
   The amendment of Claims/Claim 1 is based on the matters described in the original specification and the like as will be described below.
   (1) The amendment of "an input interface that includes, as separate operators, a switching operator for switching between the stimulation mode and the coagulation mode, a setting operator for setting a target current value, an ON operator for starting output, and an OFF operator for stopping the output;..." (The underlined parts represent amended parts. The same applies to the following description.) is based on the description regarding the configuration of the operation unit 16 constituting the input interface in the specification/[0023] and the drawings/FIG. 1. The button, the key, the knob, and the like constituting the operation unit 16 correspond to the "operators".
   (2) The amendment of "configured to cause the output unit to start outputting of the nerve stimulation pulse signal and control a current value of the nerve stimulation pulse signal to match the target current value in a case where a setting operation of the target current value is performed through the setting operator and an output start operation is performed through the ON operator in the stimulation mode," is based on the description in [0030] to [0033] of the specification.
   (3) The amendment of "a control unit configured to ..., and cause the output unit to stop outputting the nerve stimulation pulse signal in a case where the output stop operation is performed through the OFF operator" is based on the description in the specification/[0042].
   (4) The amendment of "a change in the target current value through the input interface is prohibited during a period from the output start operation through the ON operator to the output stop operation through the OFF operator in the stimulation mode" is based on the description in the specification/[0033].
Purpose of amendment The isubject matters relating to the amendment, in particular, the subject matter that the change in the target current value through the input interface is prohibited in a period from the output start operation through the ON operator to the output stop operation through the OFF operator which is an operator different from the switching operator for switching the modes in the stimulation mode is not described in Document 1 cited in the International Search Report (see Document 1/[0040] to [0055], FIG. 5 YES in STEP 8 → STEP 9).
